# EUROPÄISCHE PATENTSCHRIFT

(11) **EP 1 107 931 B1**
(45) Veröffentlichungstag und Bekanntmachung des Hinweises auf die Patenterteilung: **05.06.2002**
(21) Anmeldenummer: 99946088.4
(22) Anmeldetag: 01.09.1999
(51) Int. Cl.: C01B 37/00, B01J 29/04

(54) **VERFAHREN ZUR HERSTELLUNG EINES TITANSILIKATES MIT RUT-STRUKTUR**
METHOD FOR PRODUCING A TITANIUM SILICATE WITH RUT STRUCTURE
PROCEDE DE PRODUCTION D'UN SILICATE DE TITANE DE STRUCTURE RUT

(30) Priorität: 01.09.1998 DE 19839792
(43) Veröffentlichungstag der Anmeldung: 20.06.2001
(73) Patentinhaber: BASF AKTIENGESELLSCHAFT, 67056 Ludwigshafen (DE)
(72) Erfinder: KLEINSORGE, Martin, D-59955 Züschen (DE); GIES, Hermann, D-45549 Sprockhövel (DE); MÜLLER, Ulrich, D-67136 Fu gönheim (DE); GROSCH, Georg, Heinrich, D-67098 Bad Dürkheim (DE); RIEBER, Norbert, D-68259 Mannheim (DE)
(74) Vertreter: Isenbruck, Günter, Dr.
(86) Internationale Anmeldenummer: EP9906434
(87) Internationale Veröffentlichungsnummer: WO0012432

(56) Entgegenhaltungen:
- EP-A- 0 568 336
- EP-A- 0 692 455
- CHEMICAL ABSTRACTS, vol. 123, no. 10, 4. September 1995 (1995-09-04) Columbus, Ohio, US; abstract no. 128343, GIES, H. ET AL: "Ab-initio structure determination of zeolite RUB-10 from low resolution X-ray powder diffraction data" XP002127177 & Z. KRISTALLOGR. (1995), 210(7), 475-80 , 1995,
- CHEMICAL ABSTRACTS, vol. 122, no. 10, 6. März 1995 (1995-03-06) Columbus, Ohio, US; abstract no. 121638, OBERHAGEMANN, U. ET AL: "Rub -10, a boron containing analog of zeolite Nu-1" XP002127178 & STUD. SURF. SCI. CATAL. (1994), 84(ZEOLITES AND RELATED MICROPOROUS MATERIALS, PT. A), 435-43 ,1994,
- CHEMICAL ABSTRACTS, vol. 126, no. 7, 17. Februar 1997 (1997-02-17) Columbus, Ohio, US; abstract no. 98357, MARLER, B. ET AL: "Template directed synthesis of the high-silica microporous materials RUB -3, RUB -4, RUB -10 and RUB -13" XP002127179 & CHEM. IND. (DEKKER) (1997), 69(SYNTHESIS OF POROUS MATERIALS), 263-282 ,1997,

## Beschreibung

Die Erfindung betrifft ein Verfahren zur Herstellung eines Titansilikates mit RUT-Struktur und dessen Verwendung als Katalysator sowie ein Verfahren zur Umsetzung organischer Verbindungen mit Hilfe dieses Katalysators.

Silikate als Salze der Kieselsäuren liegen meist als einheitlich gebaute, räumlich begrenzte azyklische oder zyklische bzw. räumlich unbegrenzte Silikatanionen vor, die mittels zugeordneter Metallkationen zu größeren Komplexen verbunden sind. Beispielhaft für den Bau dieser räumlich unbegrenzten Silikatanionen sind u.a. Ketten-, Band-, Schicht- und Gerüststrukturen.

Eine wichtige Gruppe unter den Gerüstsilikaten stellen die Zeolite dar. Das Raumnetzwerk dieser Zeolite ist aufgebaut aus SiO₄-Tetraedern, die über gemeinsame Sauerstoffbrücken miteinander verbunden sind. Diese Zeolite, d.h. Alumosilikate, weisen geordnete Kanal- und Käfigstrukturen auf. Die dabei auftretenden Porenöffnungen liegen im Bereich von >0,9 nm. Eine Übersicht über die verschiedenen bekannten Strukturen der Alumosilikate findet sich beispielsweise in M.W. Meier, D. H. Olson und C. Baerlocher, "Atlas of Zeolite Structure Types, 4. Auflage, Elsevier, 1996.

Neben den Alumosilikaten sind auch Materialien bekannt, in denen im Silikatgitter Titan anstelle von Silizium vorliegt. Unter diesen Materialien ist vor allem titanhaltiges Silikat mit dem MFI-Strukturtyp zu nennen. Ein solches Silikat ist beispielsweise in der US-A-4,410,501 offenbart.

Typischerweise stellt man Titansilikate mit MFI-Struktur dadurch her, daß man zunächst eine wäßrige Mischung aus einer SiO₂-Quelle und einer Titanquelle herstellt. Diese Mischung wird dann in einem Druckbehälter in Anwesenheit einer Schablonenverbindung umgesetzt. Dieses Verfahren ist beispielsweise in der US-A-4,666,692 beschrieben.

Aufgabe der Erfindung ist es, ein verbessertes Verfahren zur Herstellung von Titansilikat bereitzustellen.

Die vorliegende Erfindung betrifft ein Verfahren zur Herstellung eines Titansilikats mit RUT-Struktur, das die Schritte (i) und (ii) umfaßt:
(i) Herstellen einer Mischung aus mindestens einer SiO₂-Quelle und mindestens einer Titanquelle;
(ii) Kristallisation der Mischung aus (i) in einem Druckbehälter unter Zugabe mindestens einer Schablonenverbindung, wobei ein Kristallisationsprodukt erhalten wird,
dadurch gekennzeichnet, daß als Schablonenverbindung Amine oder Ammoniumsalze eingesetzt werden, die zur Stabilisierung von Käfigen der Silikatstruktur [4⁴5⁴6²] und [4⁴5⁶6⁵8¹] geeignet sind.

Ebenso betrifft die vorliegende Erfindung das Titansilikat mit RUT-Struktur selbst, herstellbar durch ein Verfahren, das die Schritte (i) und (ii) umfaßt:
(i) Herstellen einer Mischung aus mindestens einer SiO₂-Quelle und mindestens einer Titanquelle;
(ii) Kristallisation der Mischung aus (i) in einem Druckbehälter unter Zugabe mindestens einer Schablonenverbindung, wobei eine Suspension erhalten wird,
dadurch gekennzeichnet, daß als Schablonenverbindung Amine oder Ammoniumsalze eingesetzt werden, die zur Stabilisierung von Käfigen der Silikatstruktur [4⁴5⁴6²] und [4⁴5⁶6⁵8¹] geeignet sind.

Als SiO₂-Quelle im oben genannten Verfahren werden vor allem Ester der Orthokieselsäure verwendet. Bevorzugt werden Tetraester eingesetzt. Besonders bevorzugt im erfindungsgemäßen Verfahren kommt Tetraethylorthosilikat zum Einsatz.

Als Titanquelle im erfindungsgemäßen Verfahren kann beispielsweise Titandioxid verwendet werden. Bevorzugt werden jedoch Titanate, besonders bevorzugt Orthotitanate und insbesondere Tetraisopropylorthotitanat verwendet.

Selbstverständlich ist es im Rahmen des erfindungsgemäßen Verfahrens möglich, auch zwei oder mehr geeigneter SiO₂-Quellen und/oder zwei oder mehr geeignete Titanquellen einzusetzen.

Aus der mindestens einen SiO₂-Quelle und der mindestens einen Titanquelle wird im erfindungsgemäßen Verfahren eine Mischung hergestellt. Besonders bevorzugt ist dabei eine wäßrige Mischung dieser Komponenten. Die Reihenfolge, in der die Komponenten gemischt werden, ist dabei unkritisch. Ebenso unkritisch ist die Art und Weise, wie die Komponenten miteinander vermischt werden. Sämtliche aus dem Stand der Technik bekannten Vorrichtungen bzw. Verfahren, wie z.B. Blattrührer, können hierbei eingesetzt werden.

Zu der oben beschriebenen Mischung wird im erfindungsgemäßen Verfahren eine Schablonenverbindung, wie oben beschrieben, zugegeben. Diese Schablonenverbindung wird bevorzugt in einer wäßrigen Lösung zur oben beschriebenen Mischung gegeben. Im allgemeinen ist die Konzentration dieser Lösung an Schablonenverbindung beliebig wählbar. Bevorzugt weist sie jedoch einen Gehalt an der Schablonenverbindung auf, der im Bereich von 1 bis 25 Gew.-%, besonders bevorzugt im Bereich von 2 bis 15 Gew.-% und insbesondere im Bereich von 3 bis 8 Gew.-% liegt.

Als Beispiele für solche Schablonenverbindungen sind u.a. Tetramethylammoniumhydroxid oder Pyrrolidin zu nennen.

Zusätzlich zu der Schablonenverbindung können im erfindungsgemäßen Verfahren auf noch eine oder mehrere weitere basische Verbindungen zu der oben genannten Mischung aus der mindestens einen SiO₂-Quelle und der mindestens einen Titanquelle hinzugefügt werden, wie z.B. Hydroxide von Ammoniumsalzen.

Der (Erd)alkalimetallgehalt des aus Schritt (ii) erhaltenen Suspension liegt im erfindungsgemäßen Verfahren bei i.a. < 1000 ppm, bevorzugt bei < 500 ppm und besonders bevorzugt bei < 200 ppm.

Je nach Wahl der mindestens einen SiO₂- und/oder Titanquelle fällt beim Herstellen der Mischung, wie oben beschrieben, gegebenenfalls aus Hydrolyse gebildeter Alkohol an. Dieser wird in der Regel bei 90 bis 100°C aus der Mischung abdestilliert, kann jedoch auch in dieser verbleiben. Der verbleibende Rückstand wird in einen Druckbehälter überführt. Sollten die Edukte so gewählt sein, daß keine Destillation erforderlich ist, kann die Mischung aus der mindestens einen SiO₂-Quelle und der mindestens einen Titanquelle sofort in den Druckbehälter überführt werden.

Bei einer Reaktionstemperatur im Bereich von im allgemeinen 80 bis 300 °C, bevorzugt 120 bis 250 °C, besonders bevorzugt 150 bis 220 °C wird die Mischung im Druckbehälter umgesetzt. Die Reaktionszeit liegt dabei im Bereich von im allgemeinen 3 bis 15, bevorzugt im Bereich von 6 bis 13, besonders bevorzugt im Bereich von 8 bis 11 Tagen.

Das aus der Umsetzung resultierende kristalline Produkt kann nach Beendigung der Reaktion nach allen gängigen Verfahren des Standes der Technik von der flüssigen Phase abgetrennt werden. Je nach Verwendungszweck des Produktes kann es notwendig sein, mit Wasser ein oder mehrere Male nachzuwaschen. Der erhaltene Feststoff wird im erfindungsgemäßen Verfahren getrocknet. Auch hier kann wiederum auf alle gängigen Verfahren des Standes der Technik zurückgegriffen werden. Beispielsweise kann der erhaltene Feststoff bei Temperaturen im Bereich von 105 bis 115°C in einem hierfür geeigneten Ofen getrocknet werden. Die Trocknungszeit beträgt dabei im allgemeinen 5 bis 20 h, bevorzugt 7 bis 15 h.

Ebenso ist es natürlich denkbar, daß das kristalline Produkt, wie oben beschrieben, das suspendiert in der flüssigen Phase vorliegt, durch Sprühtrocknung in mindestens einem Sprühschritt getrocknet wird.

Zur Entfernung der in Schritt (ii) zugegebenen Schablonenverbindung und der gegebenenfalls weiteren basischen Verbindungen wird das kristalline Produkt im Anschluß an die Trocknung mindestens einmal kalziniert.

Die Temperaturen, die bei der mindestens einen Kalzinierung gewählt werden, liegen im Bereich von im allgemeinen 120 bis 850°C, bevorzugt 180 bis 700°C, besonders bevorzugt 250 bis 550°C. Die Kalzinierung erfolgt in der Regel in sauerstoffhaltiger Atmosphäre, wobei deren Sauerstoffgehalt 0,1 bis 90 Vol.-%, bevorzugt 0,2 bis 22 Vol.-%, besonders bevorzugt 0,2 bis 10 Vol.-% beträgt. Der Druckbereich, der bei der Kalzinierung gewählt wird, liegt im allgemeinen im Bereich von 0,01 bis 5 bar, bevorzugt 0,05 bis 1,5 bar.

Demgemäß betrifft die vorliegende Erfindung auch ein Verfahren zur Herstellung eines Titansilikates mit RUT-Struktur, das zusätzlich zu den Schritten (i) und (ii), wie oben beschrieben, die Schritte (iii) und (iv) umfaßt:
(iii) Trocknung des aus (ii) resultierenden Kristallisationsprodukts;
(iv) Kalzinieren des getrockneten Produktes aus (iii).

Zur Herstellung des Titansilikates mit RUT-Struktur nach dem erfindungsgemäßen Verfahren, wie oben beschrieben, werden die Konzentrationen der mindestens einen SiO₂-Quelle und der mindestens einen Titanquelle zur Herstellung der Mischung (i) so gewählt, daß das kristalline Produkt, das aus Schritt (ii) oder (iv) resultiert, Titan in einer Konzentration enthält, die im allgemeinen im Bereich von 0,001 bis 5 Gew.-% liegt. Bevorzugt werden jedoch Titankonzentrationen im Titansilikat mit RUT-Struktur gewählt, die im Bereich von 0,002 bis 1 Gew.-%, besonders bevorzugt von 0,003 bis 0,5 Gew.-%, weiter besonders bevorzugt von 0,004 bis 0,1 Gew.-%, insbesondere bevorzugt von 0,005 bis 0,05 Gew.-% und vorzugsweise bei ungefähr 0,01 Gew.-% liegen.

Demgemäß betrifft die vorliegende Erfindung auch ein Titansilikat mit RUT-Struktur, dadurch gekennzeichnet, daß dessen Titangehalt im Bereich von 0,001 bis 5 Gew.-% liegt.

Dabei beziehen sich diese Angaben zum Titangehalt auf Ergebnisse, die aus naßchemischer Analyse erhalten werden.

Die vorliegende Erfindung betrifft auch ein Titansilikat mit RUT-Struktur, dadurch gekennzeichnet, daß es mindestens die folgenden Reflexe im Röntgendiffraktogramm aufweist:

| **Beugungswinkel 2**^{**θ**} | **Netzebenenabstand d-Wert (0,1nm)** |
|---|---|
| 10,79 | 8,19 |
| 13,69 | 6,45 |
| 14,48 | 6,10 |
| 20,19 | 4,49 |
| 22,16 | 4,00 |
| 23,26 | 3,82 |
| 27,45 | 3,24 |

Ein weiteres Charakteristikum, das titanhaltige Zeolite von Zeoliten unterscheidet, die kein Titan enthalten, ist eine spezifische Gerüstschwingungsbande im IR-Spektrum (DE 3047798). Demgemäß betrifft die vorliegende Erfindung auch ein Titansilikat mit RUT-Struktur, dadurch gekennzeichnet, daß es im IR-Spektrum eine Bande aufweist, die im Bereich von 955 bis 970 cm⁻¹ liegt.

Von Titanzeoliten mit MFI-Struktur ist bekannt, daß sie sich als Katalysatoren zur Umsetzung von organischen Verbindungen eignen. Dies ist beispielsweise in B. Notari, Stud. Surf. Sci. Catal., Vol. 37, Amsterdam, Seiten 413 bis 425 (1987) offenbart. Auch bei den erfindungsgemäßen Titansilikaten mit RUT-Struktur hat es sich gezeigt, daß sie sich als Katalysatoren eignen.

Daher betrifft die vorliegende Erfindung auch die Verwendung eines Titansilikates, wie hierin definiert, als Katalysator.

Bei der Verwendung des erfindungsgemäßen Titansilikates mit RUT-Struktur als Katalysator sind vor allem Verfahren zu nennen, in denen organische Verbindungen umgesetzt werden. Daher betrifft die vorliegende Erfindung auch ein Verfahren zur Umsetzung einer organischen Verbindung, dadurch gekennzeichnet, daß die organische Verbindung während der Umsetzung mit einem erfindungsgemäßen Katalysator, wie oben beschrieben, in Kontakt gebracht wird.

Insbesondere betrifft die vorliegende Erfindung auch ein Verfahren, dadurch gekennzeichnet, daß die organische Verbindung während der Umsetzung oxidiert wird.

Als Umsetzung sind beispielsweise zu nennen:
die Epoxidation von Olefinen wie z.B. die Herstellung von Propenoxid aus Propen und H₂O₂ oder aus Propen und Gemischen, die H₂O₂ in situ liefern;
Hydroxylierungen wie z.B. die Hydroxylierung mono-, bi- oder polycyclischer Aromaten zu mono-, di- oder höhersubstituierten Hydroxyaromaten, beispielsweise die Umsetzung von Phenol und H₂O₂ oder von Phenol und Gemischen, die H₂O₂ in situ liefern, zu Hydrochinon;
die Umwandlung von Alkanen zu Alkoholen, Aldehyden und Säuren;
die Oximbildung aus Ketonen unter Anwesenheit von H₂O₂ oder Gemischen, die H₂O₂ in situ liefern, und Ammoniak (Ammonoximierung), beispielsweise die Herstellung von Cyclohexanonoxim aus Cyclohexanon;
Isomerisierungsreaktionen wie z.B. die Umwandlung von Epoxiden zu Aldehyden,
sowie weitere in der Literatur beschriebene Umsetzungen, wie sie beispielsweise von W. Hölderich in "Zeolites": Catalysts for the Synthesis of Organic Compounds", Elsevier, Stud. Surf. Sci. Catal., 49, Amsterdam (1989), S. 69 bis 93 und insbesondere für mögliche Oxidationsreaktionen von B. Notari in Stud. Surf. Sci. Catal., 37 (1987), S. 413 bis 425, oder in Advances in Catalysis, Vol. 41, Academic Press (1996), S. 253 bis 334 beschrieben sind.

In Abhängigkeit von der Verfahrensart, in der das erfindungsgemäße Titansilikat mit RUT-Struktur als Katalysator verwendet wird, wird das Silikat entweder als Pulver oder als Formkörper eingesetzt.

Bei der Verwendung als Pulver kann direkt auf das kristalline Produkt zurückgegriffen werden, das aus dem erfindungsgemäßen Verfahren, wie oben beschrieben, resultiert.

Wird das kristalline Produkt zu einem Formkörper verformt, kann z.B. auf das getrocknete kristalline Produkt aus dem oben beschriebenen Schritt (iii) zurückgegriffen werden.

Das beispielsweise aus einem Sprühschritt erhaltene Produkt wird zur Herstellung des Formkörpers in einem weiteren Schritt des erfindungsgemäßen Verfahrens verdichtet. Dieser Verarbeitungsschritt kann in allen dafür bekannten Apparaturen erfolgen, wobei jedoch Kneter, Koller oder Extruder bevorzugt sind. Besonders bevorzugt für den großtechnischen Einsatz des erfindungsgemäßen Verfahrens wird ein Koller verwendet.

In diesem Verformungsschritt können zusätzlich eine oder mehrere viskositätssteigernde Substanzen als Anteigungsmittel zugegeben werden. Dafür können alle geeigneten, aus dem Stand der Technik bekannten Substanzen verwendet werden. Im erfindungsgemäßen Verfahren werden bevorzugt Wasser sowie Mischungen von Wasser mit einer oder mehreren organischen Substanzen, sofern diese mit Wasser mischbar sind, als Anteigungsmittel verwendet. Das Anteigungsmittel kann beim späteren Kalzinieren des Formkörpers wieder entfernt werden.

Vorzugsweise werden organische, insbesondere hydrophile organische Polymere, wie z.B. Cellulose, Cellulosederivate, wie beispielsweise Methylcellulose, Polyvinylpyrolidon, Ammonium(meth)acrylate, Tylose, besonders bevorzugt Methylcellulose verwendet.

Als weitere Zusatzstoffe können Ammoniak, Amine oder aminartige Verbindungen, wie z.B. Tetraalkylammoniumverbindungen oder Aminoalkoholate zugesetzt werden. Derartige weitere Zusatzstoffe sind in der EP-A 0389041, der EP-A 02002660 und der WO 95/19222 beschrieben, die diesbezüglich vollumfänglich in den Kontext der vorliegenden Anmeldung durch Bezugnahme einbezogen werden.

Statt basischer Zusatzstoffe ist es auch möglich, saure Zusatzstoffe zu verwenden. Bevorzugt sind organische saure Verbindungen, die sich nach dem Verformungsschritt durch Kalzinieren ausbrennen lassen. Besonders bevorzugt sind Carbonsäuren.

Zur Beeinflussung von Eigenschaften des Formkörpers kann man weitere Substanzen, vorzugsweise organische Verbindungen, insbesondere organische Polymere als weitere Zusatzstoffe zugeben, die auch die Verformbarkeit der Masse beeinflussen können. Solche Zusatzstoffe sind u.a. Alginate, Polyvinylpyrolidone, Stärke, Cellulose, Polyether, Polyester, Polyamide, Polyacrylate, Polymethylacrylate, Polyethylenimine oder Polyetherole.

Selbstverständlich können auch Gemische aus zwei oder mehr der oben genannten Zusatzstoffe eingebaut werden.

Die Zugabenreihenfolge der Zusatzstoffe ist unkritisch.

Wahlweise kann vor der Verdichtung das in der Regel noch pulverförmige Gemisch 10 bis 180 min im Kneter oder Extruder homogenisiert werden. Dabei wird in der Regel bei Temperaturen im Bereich von ungefähr 10°C bis zum Siedepunktes des Anteigungsmittel und Normaldruck oder leichtem überatmosphärischen Druck gearbeitet. Das Gemisch wird solange geknetet, bis eine verstrangbare oder extrudierfähige Masse entstanden ist.

Prinzipiell können für die Knetung und die Verformung alle herkömmlichen Knet- und Verformungsvorrichtungen bzw. Verfahren, wie sie zahlreich aus dem Stand der Technik bekannt und für die Herstellung von z.B. Katalysator-Formkörpern geeignet sind, verwendet werden.

Vorzugsweise werden Verfahren verwendet, bei denen die Verformung durch Extrusion in üblichen Extrudern, beispielsweise zu Strängen mit einem Durchmesser von üblicherweise ungefähr 1 bis ungefähr 10 mm, insbesondere ungefähr 1,5 bis ungefähr 5 mm erfolgt. Derartige Extrusionsvorrichtungen werden beispielsweise in Ullmanns "Enzyklopädie der Technischen Chemie", 4. Auflage, Bd. 2 (1972), S. 295 ff. beschrieben. Neben der Verwendung eines Extruders wird ebenfalls vorzugsweise eine Strangpresse verwendet. Im Falle einer großtechnischen Anwendung des Verfahrens wird besonders bevorzugt mit Extrudern gearbeitet.

Die Extrudate sind entweder Stränge oder Wabenkörper. Die Form der Waben ist beliebig. Es kann sich dabei beispielsweise um Rundstränge, Hohlstränge oder sternförmige Stränge handeln. Auch der Durchmesser der Waben ist beliebig. Über die äußere Form sowie den Durchmesser entscheiden in der Regel die prozeßtechnischen Anforderungen, die durch das Verfahren, in dem der Formkörper eingesetzt werden soll, vorgegeben werden.

Vor, während oder nach dem Formgebungsschritt können auf das Material Edelmetalle in Form geeigneter Edelmetallkomponenten, beispielsweise in Form von wasserlöslichen Salzen aufgebracht werden. Vorzugsweise sind dabei Katalysatoren erhältlich, die einen Gehalt von 0,01 bis 30 Gew.-% an einem oder mehreren Edelmetallen aus der Gruppe Ruthenium, Rhodium, Palladium, Osmium, Iridium, Platin, Rhenium, Gold und Silber aufweisen.

In vielen Fällen ist es jedoch am günstigsten, die Edelmetallkomponenten erst nach dem Formgebungsschritt auf die Formkörper aufzubringen, besonders dann, wenn eine Hochtemperaturbehandlung des edelmetallhaltigen Katalysators unerwünscht ist. Die Edelmetallkomponenten können insbesondere durch Ionenaustausch, Imprägnierung oder Aufsprühen auf den Formkörper gebracht werden. Das Aufbringen kann mittels organischer Lösungsmittel, wäßriger ammoniakalischer Lösungen oder überkritischer Phasen wie etwa Kohlendioxid erfolgen.

Durch den Einsatz dieser vorgenannten Methoden können durchaus verschiedenartige edelmetallhaltige Katalysatoren erzeugt werden. So kann durch Aufsprühen der Edelmetallösung auf die Formkörper eine Art Schalenkatalysator erzeugt werden. Die Dicke dieser edelmetallhaltigen Schale läßt sich durch Imprägnieren deutlich vergrößern, während beim Ionenaustausch die Katalysatorpartikel weitgehend gleichmäßig über den Formkörperquerschnitt hinweg mit Edelmetall belegt werden.

Nach Beendigung des Strangpressens oder des Extrudierens werden die erhaltenen Formkörper bei im allgemeinen 50 bis 250°C, bevorzugt 80 bis 250°C bei Drücken von im allgemeinen 0,01 bis 5 bar, bevorzugt 0,05 bis 1,5 bar im Laufe von ungefähr 1 bis 20 h getrocknet.

Die anschließende Kalzinierung erfolgt bei Temperaturen von 250 bis 800°C, bevorzugt 350 bis 600°C, besonders bevorzugt 400 bis 500°C. Der Druckbereich wird ähnlich dem der Trocknung gewählt. In der Regel wird in sauerstoffhaltiger Atmosphäre kalziniert, wobei der Sauerstoffgehalt 0,1 bis 90 Vol.-%, bevorzugt 0,2 bis 22 Vol.-%, besonders bevorzugt 0,2 bis 10 Vol.-% beträgt.

Vorzugsweise wird das erfindungsgemäße Titansilikat mit RUT-Struktur bei Verwendung als Katalysator als Pulver eingesetzt.

Insbesondere eignet sich das vorstehend ausführlich diskutierte Titansilikat mit RUT-Struktur für die Epoxidation von Alkenen. Demgemäß betrifft die vorliegende Erfindung auch ein Verfahren, daß dadurch gekennzeichnet ist, daß ein Alken zu einem Alkenoxid umgesetzt wird.

Alkene, die für eine solche Funktionalisierung in Frage kommen, sind beispielsweise

Ethylen, Propylen, But-1-en, But-2-en, Isobuten, Butadien, Pentene, Iso- a-ymlen, Piperylen, Hexene, Hexadiene, Heptene, Octene, Diisobuten, Trimethylpenten, Nonene, Dodecen, Tridecen, Tetradecene bis Eicosene, Tri- und Tetrapropylen, Polybutadiene, Polyisobutene, Isoprene, Terpene, Geraniol, Linalol, Linalylacetat, Methylencyclopropan, Cyclopenten, Cyclohexen, Norbornen, Cyclohepten, Vinylcyclohexan, Vinyloxiran, Vinylcyclohexen, Styrol, Cycloocten, Cyclooctadien, Vinylnorbornen, Inden, Tetrahydroinden, Methylstyrol, Dicyclopentadien, Divinylbenzol, Cyclododecen, Cyclododecatrien, Stilben, Diphenylbutadien, Vitamin A, Beta-Carotin, Vinylidenfluorid, Allylhalogenide, Crotylchlorid, Methallylchlorid, Dichlorbutene, Allylalkohol, Methallylalkohol, Butenole, Butendiole, Cyclopentendiole, Pentenole, Octadienole, Tridecenole, ungesättigte Steroide, Ethoxyethylen, Isoeugenol, Anethol, Isoallesafrol, ungesättigte Carbonsäuren wie etwa Acrylsäure, Methacrylsäure, Crotonsäure, Maleinsäure, Vinylessigsäure, ungesättigte Fettsäuren wie etwa Ölsäure, Linolsäure, Palmitinsäure, sowie natürlich vorkommende Fette und Öle.

Ein Vorteil des erfindungsgemäß hergestellten Titansilikates mit RUT-Struktur liegt darin begründet, daß das kristalline Produkt, das, wie oben beschrieben, aus dem erfindungsgemäßen Verfahren resultiert, eine große spezifische externe Oberfläche aufweist. Diese liegt im allgemeinen im Bereich von 10 bis 200 m²/g, bevorzugt im Bereich von 80 bis 120 m²/g.

Diese Werte für die spezifische externe Oberfläche beziehen sich auf Ergebnisse, die über Stickstoff-Adsorption gemäß DIN 66131 erhalten werden.

Weiter weist das erfindungsgemäße Titansilikat mit RUT-Struktur eine besondere interne Struktur auf, die dadurch gekennzeichnet ist, daß im wesentlichen keine Poren mit einer Öffnungsweite von > 5,5 Å vorliegen.

Damit können, ohne die Einschränkung eines Porensystems, wie bei Zeoliten mit anderer als der RUT-Struktur, auch sterisch sehr anspruchsvolle Moleküle und/oder Naturstoffgemische an der externen Oberfläche katalytisch umgesetzt werden. Da die Katalyse am erfindungsgemäßen Titansilikat mit RUT-Struktur an der externen Oberfläche stattfindet, können höhermolekulare Verbindungen in polymeranalogen Reaktionen sämtlicher oben genannter Reaktionsklassen umgesetzt werden.

Bei der Verwendung des erfindungsgemäßen Titansilikates mit RUT-Struktur als Katalysator bei der Umsetzung von einem Alken zu einem Alkenoxid kann auf sämtliche, zu diesem Zweck geeignete Oxidationsmittel zurückgegriffen werden. Beispielsweise sind dabei Wasserstoffperoxid, Zusammensetzungen, die Wasserstoffperoxid in situ erzeugen können, oder organische Hydroperoxide zu nennen.

Ein Vorteil des erfindungsgemäßen Titansilikates mit RUT-Struktur bei Verwendung als Katalysator ist darin zu sehen, daß im Gegensatz zu den titanhaltigen Zeolitkatalysatoren gemäß dem Stand der Technik beispielsweise Wasserstoffperoxidlösungen verwendet werden können, die eine sehr niedrige Konzentration an H₂O₂ aufweisen.

Bevorzugt werden H₂O₂-Lösungen eingesetzt, deren Konzentration an H₂O₂ im Bereich von 0,05 bis 40 Gew.-%, besonders bevorzugt von 0,1 bis 20 Gew.-%, insbesondere von 0,5 bis 10 Gew.-% liegt.

Insbesondere in dem Fall, in dem als Oxidationsmittel ein Gemisch aus Wasserstoff und Sauerstoff verwendet wird, kann das erfindungsgemäße Titansilikat mit RUT-Struktur zusätzlich zu Titan, Silizium und Sauerstoff auch ein oder mehrere Elemente umfassen, ausgewählt aus der Gruppe bestehend aus Ruthenium, Rhodium, Palladium, Osmium, Iridium, Platin, Eisen, Kobalt, Nickel, Rhenium, Silber und Gold.

Natürlich ist es im Rahmen des erfindungsgemäßen Verfahrens auch möglich, das als Katalysator eingesetzte Titansilikat mit RUT-Struktur nach dessen Verbrauch zu regenerieren.

Wurde der Katalysator in Pulverform eingesetzt, kann er zur Regenerierung beispielsweise mit oxidierenden Mineralsäuren, wie etwa Salpetersäure, gewaschen und im Anschluß daran mit Wasserstoffperoxid im Rückfluß gekocht werden.

Wurde das erfindungsgemäße Titansilikat mit RUT-Struktur als Formkörper-Katalysator verwendet, so kann der Formkörper zur Regenerierung in oder außerhalb der verwendeten Reaktionsanordnung, wie z.B. einem Reaktor, mit Sauerstoff enthaltenden oder liefernden Gasen, wie z.B. Luft, synthetische Luft, Stickoxide sowie molekularem Sauerstoff beaufschlagt werden. Dabei wird der Katalysator vorzugsweise ausgehend von Raumtemperatur auf Temperaturen von 120 bis 850°C, bevorzugt 180 - 700°C und besonders bevorzugt 250 - 550°C aufgeheizt, wobei man einem über dem Katalysator strömenden Inertgas Luft oder Sauerstoff in Konzentrationen von im allgemeinen 0,1 bis 90 Vol.-%, bevorzugt 0,2 bis 22 Vol.-% besonders bevorzugt 0,2 bis 10 Vol.-%, bezogen auf dem Gesamtgasstrom, zusetzt. Dabei wird bei einem Druck von im allgemeinen 0,01 bis 5 bar, bevorzugt 0,05 bis 1,5 gearbeitet.

Die nachstehenden Beispiele sollen das erfindungsgemäße Verfahren näher erläutern, ohne daß dadurch eine Einschränkung zu verstehen wäre.

### BEISPIELE

### Beispiel 1

In einem Vierhalskolben (21 Inhalt) wurden 455 g Tetraethylorthosilikat vorgelegt und aus einem Tropftrichter innerhalb von 30 min mit 15 g Tetraisopropylorthotitanat unter Rühren (250 U/min, Blattrührer) versetzt. Es bildete sich eine farblose, klare Mischung. Abschließend versetzte man mit 800 g einer 20 Gew.-%-igen Tetramethylammoniumhydroxid-Lösung (Alkaligehalt <10 ppm) und rührte noch eine Stunde nach. Bei 90 bis 100°C wurde das aus der Hydrolyse gebildete Alkoholgemisch (ca. 450 g) abdestilliert. Man füllte mit 1,5 l deionisiertem Wasser auf und gab das mittlerweile leicht opaque Sol in einen 2,5 l fassenden Rührautoklaven (Edelstahl 1.4571).

Mit einer Heizrate von 3°/min wurde der verschlossene Autoklav (Ankerrührer, 200 U/min) auf eine Reaktionstemperatur von 175°C gebracht. Nach 10 Tagen wurde die Reaktion beendet. Das erkaltete Reaktionsgemisch wurde abzentrifugiert und mehrfach mit Wasser neutralgewaschen. Der erhaltene Feststoff wurde bei 100°C innerhalb von 24 Stunden getrocknet (Auswaage 149 g).

Abschließend wurde unter Luft bei 550°C in 5 Stunden das im Produkt noch verbliebene Templat abgebrannt (Kalzinierungsverlust: 14 Gew.-%).

Das gebrannte Produkt hatte nach naßchemischer Analyse eine Ti-Gehalt von 1,5 Gew.-% und einen Gehalt an Restalkali unterhalb 100 ppm. Die Ausbeute auf eingesetztes SiO₂ betrug 87 %. Die Kristallite hatten eine Größe von 0,05 bis 0,25 Um und das Produkt zeigte im IR eine typische Bande bei ca. 960 cm⁻¹.

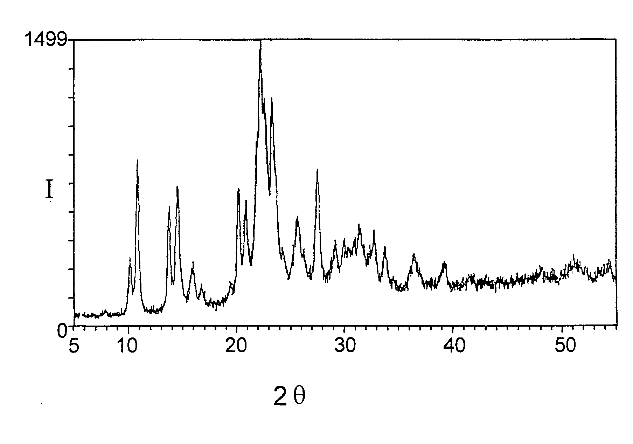

Das Produkt zeigt das in Abbildung 1 wiedergegebene Röntgendiffraktogramm. In Abbildung 1 ist auf der Ordinate die Intensität I aufgetragen.

### Beispiel 2

In einen 250 ml Glasautoklaven wurden 36 g Methanol und 0,5 g Titansilikatpulver aus Beispiel 1 eingefüllt und die Suspension mit einem Magnetrührer gerührt. Der verschlossene Glasautoklav wurde danach auf -30°C abgekühlt. Dann wurden 10 g Propen aufgepreßt. Danach wurde der Glasautoklav auf 0°C erwärmt und 17 g einer 30 %-igen Wasserstoffperoxidlösung zudosiert. Die Reaktionsmischung wird 5 h bei 0°C unter Eigendruck gerührt. Danach wurde der Katalysator abzentrifugiert und der Gehalt an Propylenoxid gaschromatographisch bestimmt. Der Gehalt an Propylenoxid betrug 0,3 Gew.-%.

### Beispiel 3

In einen 250 ml Glasautoklaven wurden 36 ml Methanol und 0,5 g Titansilikat aus Beispiel 1 eingefüllt und die Suspension mit einem Magnetrührer gerührt. Der verschlossene Glasautoklav wurde danach auf -30°C abgekühlt und 20,2 g Propen aufgepreßt. Danach wurde der Glasautoklav auf 0°C erwärmt und 23 g 0,5 %-ige Wasserstoffperoxidlösung zudosiert. Die Reaktionsmischung wurde 30 min bei 0°C unter Eigendruck gerührt. Danach wurde der Katalysator abzentrifugiert und der Gehalt an Propylenoxid gaschromatographisch bestimmt. Der Gehalt an Propylenoxid betrug 0,098 Gew.-%.

## Patentansprüche

1. Verfahren zur Herstellung eines Titansilikats mit RUT-Struktur, das die Schritte (i) und (ii) umfaßt:
(i) Herstellen einer Mischung aus mindestens einer SiO₂-Quelle und mindestens einer Titanquelle;
(ii) Kristallisation der Mischung aus (i) in einem Druckbehälter unter Zugabe mindestens einer Schablonenverbindung, wobei eine Suspension erhalten wird,
**dadurch gekennzeichnet, daß** als Schablonenverbindung Amine oder Ammoniumsalze eingesetzt werden, die zur Stabilisierung von Käfigen der Silikatstruktur [4⁴5⁴6²] und [4⁴5⁶6⁵8¹] geeignet sind.

2. Verfahren nach Anspruch 1, **dadurch gekennzeichnet, daß** es zusätzlich die Schritte (iii) und (iv) umfaßt:
(iii) Trocknung des aus (ii) resultierenden Kristallisationsproduktes;
(iv) Kalzinieren des getrockneten Produktes aus (iii).

3. Titansilikat mit RUT-Struktur, herstellbar durch ein Verfahren, das die Schritte (i) und (ii) umfaßt:
(i) Herstellen einer Mischung aus mindestens einer SiO₂-Quelle und mindestens einer Titanquelle;
(ii) Kristallisation der Mischung aus (i) in einem Druckbehälter unter Zugabe mindestens einer Schablonenverbindung, wobei eine Suspension erhalten wird,
**dadurch gekennzeichnet, daß** als Schablonenverbindung Amine oder Ammoniumsalze eingesetzt werden, die zur Stabilisierung von Käfigen der Silikatstruktur [4⁴5⁴6²] und [4⁴5⁶6⁵8¹] geeignet sind.

4. Titansilikat mit RUT-Struktur nach Anspruch 3, **dadurch gekennzeichnet, daß** dessen Titangehalt im Bereich von 0,001 bis 5 Gew.-% liegt.

5. Titansilikat mit RUT-Struktur nach Anspruch 3 oder 4, **dadurch gekennzeichnet, daß** es mindestens die folgenden Reflexe im Röntgendiffraktogramm aufweist:
| **Beugungswinkel 2**^{**θ**} | **Netzebenenabstand d-Wert (0,1nm)** |
|---|---|
| 10,79 | 8,19 |
| 13,69 | 6,45 |
| 14,48 | 6,10 |
| 20,19 | 4,49 |
| 22,16 | 4,00 |
| 23,26 | 3,82 |
| 27,45 | 3,24 |

6. Titansilikat mit RUT-Struktur nach einem der Ansprüche 3 bis 5, **dadurch gekennzeichnet, daß** es im IR-Spektrum eine Bande aufweist, die im Bereich von 955 bis 970 cm⁻¹ liegt.

7. Verwendung eines gemäß Anspruch 1 oder 2 hergestellten Titansilikats mit RUT-Struktur oder eines Titansilikats mit RUT-Struktur gemäß einem der Ansprüche 3 bis 6 als Katalysator.

8. Verfahren zur Umsetzung einer organischen Verbindung, **dadurch gekennzeichnet, daß** die organische Verbindung während der Umsetzung mit einem Titansilikat gemäß einem der Ansprüche 3 bis 6 in Kontakt gebracht wird.

9. Verfahren nach Anspruch 8, **dadurch gekennzeichnet, daß** die organische Verbindung während der Umsetzung oxidiert wird.

10. Verfahren nach Anspruch 8 oder 9, **dadurch gekennzeichnet, daß** ein Alken zu einem Alkenoxid umgesetzt wird.

## Claims

1. A process for preparing a titanium silicate having the RUT structure, which comprises the steps (i) and (ii):
(i) preparation of a mixture comprising at least one SiO₂ source and at least one titanium source;
(ii) crystallization of the mixture from (i) in a pressure vessel with addition of at least one template compound to give a crystallization product,
wherein the template compounds used are amines or ammonium salts which are suitable for stabilizing cages of the silicate structure [4⁴5⁴6²] and [4⁴5⁶6⁵8¹].

2. A process as claimed in claim 1 which additionally comprises the steps (iii) and (iv):
(iii) drying of the crystallization product resulting from (ii);
(iv) calcination of the dried product from (iii).

3. A titanium silicate having the RUT structure, able to be prepared by a process comprising the steps (i) and (ii):
(i) preparation of a mixture comprising at least one SiO₂ source and at least one titanium source;
(ii) crystallization of the mixture from (i) in a pressure vessel with addition of at least one template compound to give a crystallization product,
wherein the template compounds used are amines or ammonium salts which are suitable for stabilizing cages of the silicate structure [4⁴5⁴6²] and [4⁴5⁶6⁵8¹].

4. A titanium silicate having the RUT structure as claimed in claim 3 whose titanium content is in the range from 0.001 to 5% by weight.

5. A titanium silicate having the RUT structure as claimed in claim 3 or 4 which has at least the following reflections in the X-ray diffraction pattern:
| **Diffraction angle 2θ** | **Lattice plane spacing d (0.1 nm)** |
|---|---|
| 10.79 | 8.19 |
| 13.69 | 6.45 |
| 14.48 | 6.10 |
| 20.19 | 4.49 |
| 22.16 | 4.00 |
| 23.26 | 3.82 |
| 27.45 | 3.24 |

6. A titanium silicate having the RUT structure as claimed in any of claims 3 to 5 which has a band in the range from 955 to 970 cm⁻¹ in the IR spectrum.

7. The use of a titanium silicate having the RUT structure prepared as claimed in claim 1 or 2 or a titanium silicate having the RUT structure as claimed in any of claims 3 to 6 as catalyst.

8. A process for the reaction of an organic compound, which comprises bringing the organic compound into contact with a titanium silicate as claimed in any of claims 3 to 6 during the reaction.

9. A process as claimed in claim 8, wherein the organic compound is oxidized during the reaction.

10. A process as claimed in claim 8 or 9, wherein an alkene is reacted to form an alkene oxide.

## Revendications

1. Procédé de fabrication d'un silicate de titane à structure RUT, qui comprend les étapes (i) et (ii) ci-après consistant à :
(i) préparer un mélange à partir d'au moins une source de SiO₂ et d'au moins une source de titane ;
(ii) cristalliser le mélange obtenu sous (i) dans un récipient sous pression en ajoutant au moins un composé de gabarit pour obtenir une suspension,
**caractérisé en ce qu'**on met en oeuvre à titre de composé de gabarit, des amines ou des sels d'ammonium qui sont appropriés pour la stabilisation de cages de la structure de silicate [4⁴5⁴6²] et [4⁴5⁶6⁵8¹].

2. Procédé selon la revendication 1, **caractérisé en ce qu'**il comprend en outre les étapes (iii) et (iv) consistant à :
(iii) sécher le produit de cristallisation résultant de (ii) ;
(iv) calciner le produit séché provenant de (iii).

3. Silicate de titane à structure RUT que l'on peut préparer via un procédé qui comprend les étapes (i) et (ii) ci-après consistant à :
(i) préparer un mélange à partir d'au moins une source de SiO₂ et d'au moins une source de titane ;
(ii) cristalliser le mélange obtenu sous (i) dans un récipient sous pression en ajoutant au moins un composé de gabarit pour obtenir une suspension,
**caractérisé en ce qu'**on met en oeuvre à titre de composé de gabarit, des amines ou des sels d'ammonium qui sont appropriés pour la stabilisation de cages de la structure de silicate [4⁴5⁴6²] et [4⁴5⁶6⁵8¹].

4. Silicate de titane à structure RUT selon la revendication 3, **caractérisé en ce que** sa teneur en titane se situe dans la plage de 0,001 à 5 % en poids.

5. Silicate de titane à structure RUT selon la revendication 3 ou 4,
**caractérisé en ce qu'**il présente au moins les réflexions ci-après dans le diffractogramme des rayons X :
| Angle de diffraction 2^{θ} | Valeur d de l'écartement des plans (0,1 nm) |
|---|---|
| 10,79 | 8,19 |
| 13,69 | 6,45 |
| 14,48 | 6,10 |
| 20,19 | 4,49 |
| 22,16 | 4,00 |
| 23,26 | 3,82 |
| 27,45 | 3,24 |

6. Silicate de titane à structure RUT selon l'une quelconque des revendications 3 à 5, **caractérisé en ce qu'**il présente, dans le spectre infrarouge, une bande qui se situe dans la plage de 955 à 970 cm⁻¹.

7. Utilisation d'un silicate de titane à structure RUT préparé conformément à la revendication 1 ou 2 ou d'un silicate de titane à structure RUT selon l'une quelconque des revendications 3 à 6, comme catalyseur.

8. Procédé pour la mise en réaction d'un composé organique, **caractérisé en ce qu'**on amène le composé organique, au cours de la mise en réaction, en contact avec un silicate de titane selon l'une quelconque des revendications 3 à 6.

9. Procédé selon la revendication 8, **caractérisé en ce qu'**on oxyde le composé organique lors de la mise en réaction.

10. Procédé selon la revendication 8 ou 9, **caractérisé en ce qu'**on fait réagir un alcène pour obtenir un oxyde d'alcène.
